# EUROPÄISCHE PATENTSCHRIFT

(11) **EP 0 826 363 B1**
(45) Veröffentlichungstag und Bekanntmachung des Hinweises auf die Patenterteilung: **13.02.2002**
(21) Anmeldenummer: 97114429.0
(22) Anmeldetag: 21.08.1997
(51) Int. Cl.: A61K 7/42

(54) **Kosmetisches Mittel mit polymergebundenen Benzophenonchromophoren**
Cosmetics with benzophenones chromophores bound on polymers
Cosmétique avec des benzophenones chromophores liées à des polymères

(30) Priorität: 26.08.1996 DE 19634401
(43) Veröffentlichungstag der Anmeldung: 04.03.1998
(73) Patentinhaber: BASF AKTIENGESELLSCHAFT, 67056 Ludwigshafen (DE)
(72) Erfinder: Keller, Harald, Dr., 67069 Ludwigshafen (DE); Sperling-Vietmeier, Karin, Dr., 67433 Neustadt (DE); Westenfelder, Horst, 67435 Neustadt (DE)

(56) Entgegenhaltungen:
- US-A- 3 341 493
- US-A- 3 956 244

## Beschreibung

Die vorliegende Erfindung betrifft kosmetische Mittel mit polymergebundenen chromophoren Gruppen zum Schutz der Haut und der Haare vor UV-Strahlung.

Bei kosmetischen Sonnenschutzmitteln, die auf der Haut aufgetragen werden, ist häufig eine gute Wasserfestigkeit wünschenswert. Diese Eigenschaft wird insbesondere gewünscht, wenn häufiger Kontakt der Haut mit Wasser oder wäßrigen Flüssigkeiten wie Schweiß stattfindet. Um die gewünschte Wasserfestigkeit zu erreichen, werden solchen Mitteln häufig Polymere zugesetzt.

Eine weitere Möglichkeit besteht darin, die UV-absorbierenden Gruppen kovalent mit einem Polymer zu verknüpfen.

In WO 89/4824 werden Copolymere aus dem UV-absorbierenden Styrol und Maleinsäureanhydrid, Vinylpyrrolidon oder Acrylaten beschrieben. Diese Copolymere besitzen eine hohe Wasserlöslichkeit und eine geringe Hautpenetration.

In JP 60/88066 werden UV-absorbierende Benzophenonderivate beschrieben, die über einen Urethanspacer an (Meth)acrylate gebunden sind, beschrieben. Diese Polymere eignen sich als Material für Kontaktlinsen und Brillengläser.

Neben der guten Wasserfestigkeit sollen die kosmetischen Mittel jedoch eine Reihe von anderen Eigenschaften wie geringe Hautpenetration, geringer Gehalt an Restmonomeren, die aus toxikologischen und ölfaktorischen Gründen zu vermeiden sind, gute Verarbeitbarkeit, Mischbarkeit und Stabilität mit weiteren Komponenten kosmetischer Mittel, erfüllen.

Gefunden wurden kosmetisches Mittel, enthaltend ein Polymer mit der sich wiederholenden Struktureinheit (I) 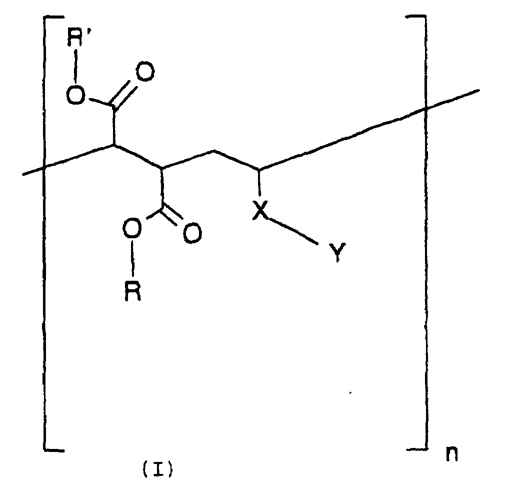 wobei
- R und R': gleich oder verschieden sein können und H, Alkalimetall, Ammonium, C₁-C₃₀ Alkyl,
- X: eine direkte Bindung, C₁-C₈ Alkylen oder C₂-C₁₂ Oxoalkylen mit 1-6 Sauerstoffatomen,
- Y: ein UV-Licht absorbierender organischer Rest,
- n: 2-800 bedeutet.

Die Löslichkeit der Polymere mit der Struktureinheit (I) läßt sich über die Natur der Reste R und R'gezielt einstellen. Wird etwa ein in Wasser oder wäßrigen Lösungsmitteln lösliches Polymer gewünscht, wird als Rest R bzw. R' bevorzugt Wasserstoff oder Alkalimetallionen, wie Natrium und Kalium oder Ammoniumionen verwendet. Der Rest R braucht nicht bei dem gesamten Polymer identisch sein, sondern kann auch aus Mischungen verschiedener Reste R und R' bestehen.

Wird beispielsweise das Polymer mit R=H teilweise mit der Base NaOH neutralisiert, so liegen - je nach Neutralisationsgrad - einige Reste als R=H und einige als R=Na vor.

Wird dagegen ein öllösliches Polymer gewünscht, wird in der Regel ein langkettiger hydrophober Rest R und R'verwendet. Bevorzugt sind dazu langkettige verzweigte oder unverzweigte Alkylreste mit 12- 30, besonders bevorzugt 16- 20 C-Atomen als Reste R und R'. Es können jedoch auch andere Reste R und R', beispielsweise ein oder mehrfach ungesättigte hydrophobe Alkenylreste verwendet werden.

Die Polymere für die erfindungsgemäßen kosmetischen Mittel lassen sich herstellen, indem man Monomere der Formel II unter den für radikalische Polymerisationen üblichen Bedingungen unter Ausschluß von Sauerstoff polymerisiert.

Als Lösungsmittel eignen sich die üblichen organischen Lösungsmittel, insbesondere Ester wie Ethylacetat und Butylacetat, Ketone wie Aceton, Methylethylketon und Cyclohexanon, Ether wie Tetrahydrofuran und Methyl-tert.Butylether und Alkane wie Hexan, Heptan, Cyclohexan und Octan. Als Starter eignen sich Azostarter wie z. B. Azoisobutyronitril und Peroxide wie z. B. Dibenzoylperoxid oder tertiär-Butyl-2-ethyl-perhexanoat. Die Reaktionstemperatur beträgt 40 bis 160°C, bevorzugt 80 bis 120°C.

Die Monomere der allgemeinen Formel (II) lassen sich durch folgende bekannte Verfahen herstellen:

### 1-Olefinether

Durch Williamson Synthese aus den entsprechenden 1-Olefinchloriden oder 1-Olefinbromiden und den OH-Gruppen tragenden UV-Licht absorbierenden Chromophoren (siehe J. March, Advanced Organic Chemistry,J . Wiley&Sons, 1985, S. 342).

### 1-Olefinester

Durch Veresterung der UV-Licht absorbierenden Chromophore mit den entsprechenden 1-Olefinalkoholen (siehe J. March, Advanced Organic Chemistry, J. Wiley&Sons, 1985, S. 348).

### Vinylester

Durch Umesterung der entsprechenden carboxylgruppen haltigen UV-Licht absorbierenden Chromophore mit Vinylacetat (siehe G. Heublein, B. Heublein, B. Heyroth, E. Brendel, Z. Chem. 19 (1979) 104).

### Vinylether

Durch Reaktion der OH-Gruppen tragenden UV-Licht absorbierenden Chromophoren mit Acetylen (siehe Organikum, Deutscher Verlag der Wissenschaften, Berlin, 1979, S. 338)

Neben den Monomeren der Formel II können weitere vinylische Comonomere wie z. B. Vinylpyrolidon, Vinylacetat, 1-Olefine, Acrylate und Methacrylate einpolymerisiert werden.

Das so erhaltene Polymer wird anschließend mit R-OH und R'-OH umgesetzt. Hierbei hat man es durch Wahl der Gruppe R in der Hand, die Löslichkeitseigenschaften des Polymeren gezielt einzustellen. Wählt man als R-OH eine wäßrige Base, so erhält man wasserlösliches Polymer. Wird das Anhydrid dagegen mit einem Fettalkohol umgesetzt, so wird das Polymer öllöslich.

Das nach R-OH Umsetzung erhaltene Polymer wird dann mit in der Kosmetik üblichen Hilfs- und Zusatzstoffen je nach Anwendungszweck formuliert. Beispiele geeigneter Hilfs-und Zusatzstoffe für Sonnenschutzmittel sind bei W. Umbach, Kosmetik, Georg-Thieme-Verlag Stuttgart, 1988 beschrieben.

Die erfindungsgemäßen kosmetischen Mittel zeichnen sich durch ihre hohe Wasserbeständigkeit, gute Hautverträglichkeit und geringe Hautpenetration aus.

Die Erfindung wird durch die folgenden Beispiele weiter veranschaulicht.

### Beispiel 1a

### Herstellung eines UV-Licht absorbierenden Polymers

3.85 g Maleinsäureanhydrid und 4 g Butylacetat werden unter Stickstoff vorgelegt und auf 100° C erwärmt. Innerhalb von 2h werden aus getrennten Gefäßen eine Lösung von 7.5 g p-Dimethylaminobenzoesäurevinylester in 18 g Butylacetat und 0.91 g t-Butylperoxy-2-ethylhexanoat in 10 g Butylacetat zudosiert. Nach Beendigung der Zugabe der beiden Lösungen wird 4h bei 100° C gerührt. Nach dem Erkalten wird die Reaktionsmischung in 300 ml t-Butyl-methylether gegossen und das ausfallende Polymer durch Filtration abgetrennt.
Ausbeute: 2,17 g
Molekulargewicht nach GPC: Mn = 1370 g/mol, Polydispersität = 1.8
UV: 314 nm , E¹₁= 624

### Beispiel 1b

### Herstellung eines öllöslichen polymeren UV-Absorbers

1.98 g des in Beispiel 1a hergestellten Polymers werden mit 3.91 g 2-Octyl-dodecanol gemischt und 6h bei 150° C gerührt. Man erhält dabei eine klare viskose Polymermasse, die 42 % nicht umgesetztes 2-Octyl-dodecanol enthält.
Molekulargewicht nach GPC: Mn = 3360 g/mol, Polydispersität = 1.3
UV: 306 nm , E¹₁= 142

### Beispiel 2

### Herstellung eines UV-Licht absorbierenden Polymers

14.84 g Maleinsäureanhydrid und 15.1 g Butylacetat werden unter Stickstoff vorgelegt und auf 100° C erwärmt. Innerhalb von 2h werden aus getrennten Gefäßen 25 g Salicylsäurevinylester und 3.19 g t-Butylperoxy-2-ethylhexanoat in 10 g Butylacetat zudosiert. Nach Beendigung der Zugabe der beiden Lösungen wird 4h bei 100° C gerührt. Nach dem Erkalten wird die Reaktionsmischung in 500 ml t-Butyl-methylether gegossen und das ausfallende Polymer durch Filtration abgetrennt.
Ausbeute: 29.6 g
Molekulargewicht nach GPC: Mn = 1600 g/mol, Polydispersität = 3.9
UV: 309 nm , E¹₁= 131

### Beispiel 3

### Herstellung eines öllöslichen polymeren UV-Absorbers

15 g des in Beispiel 2 hergestellten Polymers werden mit 32.5 g 2-Octyl-dodecanol gemischt und 14h bei 150° C gerührt. Man erhält dabei eine trübe viskose Polymermasse, die 22 % nicht umgesetztes 2-Octyl-dodecanol enthält. Nach Fällen in 250 g Ethanol erhält man einen polymeren Feststoff.
Ausbeute: 16.8 g
Molekulargewicht nach GPC: Mn = 10600 g/mol,
Polydispersität = 2.1
UV: 310 nm E¹₁= 50

### Beispiel 4

### Herstellung eines UV-Licht absorbierenden Polymers

8.47 g Maleinsäureanhydrid und 19.76 g Toluol werden unter Stickstoff vorgelegt und auf 100° C erwärmt. Innerhalb von 2h werden aus getrennten Gefäßen eine Lösung von 25 g 2,2-Diphenyl-1-cyanoacrylsäureallylester in 42 g Toluol und 0.67 g t-Butylperoxy-2-ethylhexanoat in 16 g Toluol zudosiert. Nach Beendigung der Zugabe der beiden Lösungen wird 4h bei 100° C gerührt. Nach dem Erkalten wird die Reaktionsmischung in 500 ml t-Butyl-methylether gegossen und das ausfallende Polymer durch Filtration abgetrennt.
Ausbeute: 5.8 g
Molekulargewicht nach GPC: Mn = 1620 g/mol, Polydispersität = 1.4
UV: 305 nm , E¹₁= 252

### Beispiel 5

### Herstellung eines UV-Licht absorbierenden Polymers

9.86 g Maleinsäureanhydrid und 50 g Tetrahydrofuran werden unter Stickstoff vorgelegt und auf 75° C erwärmt. Innerhalb von 1h werden aus getrennten Gefäßen eine Lösung von 25 g p-Methoxyzimtsäurevinyloxyethylester und 2.02 g 1-Dodecylthiol in 24 g Tetrahydrofuran und 0.34 g 2,2-Azobis(2-methylbutyronitril) in 10 g Tetrahydrofuran zudosiert. Nach Beendigung der Zugabe der beiden Lösungen wird 8h bei 75° C gerührt. Nach dem Erkalten wird die Reaktionsmischung in 300 ml t-Butyl-methylether gegossen und das ausfallende Polymer durch Filtration abgetrennt.
Ausbeute: 30 g
Molekulargewicht nach GPC: Mn = 2800 g/mol, Polydispersität = 2.8
UV: 310 nm, E¹₁= 292

### Beispiel 6

Herstellung eines UV-Licht absorbierenden Polymers57.8 g Maleinsäureanhydrid und 58 g Butylacetat werden unter Stickstoff vorgelegt und auf 100° C erwärmt. Innerhalb von 4h werden aus getrennten Gefäßen eine Lösung von 150 g 1-{2-Hydroxy-4-allyloxy)benzophenon in 280 g Butylacetat und 16.6 g t-Butylperoxy-2-ethylhexanoat in 10 g Butylacetat zudosiert. Nach Beendigung der zugabe der beiden Lösungen wird 2h bei 100° C gerührt. Nach dem Erkalten wird die Reaktionsmischung in 5 l t-Butyl-methylether gegossen und das ausfallende Polymer durch Filtration abgetrennt.
Ausbeute: 196.8 g
Molekulargewicht nach GPC: Mn = 1744 g/mol, Polydispersität = 1.7

### Beispiel 7

### Herstellung eines öllöslichen polymeren UV-Absorbers

100 g des in Beispiel 6 hergestellten Polymers werden mit 170 g 2-Octyl-dodecanol gemischt und 5h bei 150° C gerührt. Man erhält dabei eine klare viskose Polymermasse, die 16.5 % nicht umgesetztes 2-Octyl-dodecanol enthält.
Molekulargewicht nach GPC: Mn = 5510 g/mol, Polydispersität = 1.8
UV: 314 nm E¹₁= 92
   274 nm E¹₁= 147

### Beispiel 8

Herstellung eines wasserlöslichen polymeren UV-Absorbers
10 g des in Beispiel 6 hergestellten Polymeren werden in 100 g Wasser suspendiert. Anschließend werden 1.14 g Natriumhydroxid zugegeben und die Mischung 10 min auf 100° C erwärmt. Es entsteht eine klare gelbe Lösung. Die wässrige Polymerlösung kann mit Wasser weiter verdünnt werden.

### Beispiel 9

Die in Beispiel 8 hergestellte Polymerlösung wird durch Zugabe von 32 prozentiger Salzsäure bis pH = 2 angesäuert. Dabei fällt ein farbloses Polymer aus.
Ausbeute: 9 g

### Beispiel 10

### Herstellung eines Sonnenschutzmittels

Phase A: 18 g des in Beispiel 7 hergestellten Polymers, 5 g Finsolv TN, 10 g Witconol APM und 0.5 g Nip Nip werden bei 60° C gemischt und nach Abkühlen mit 1 g Cremophor RH40 gemischt.
Phase C: 0.3 Pemulen TR1, 5 g 1,2-Propylenglykol und 65 g Wasser werden gemischt. Danach werden 0.2 g Tylose H4000 eingerührt. Die Phase A wird in die Phase C eingerührt und durch Zugabe von 0.3 g Triethanolamin neutralisiert.

## Patentansprüche

1. Kosmetisches Mittel, enthaltend ein Polymer mit der sich wiederholenden Struktureinheit (I) 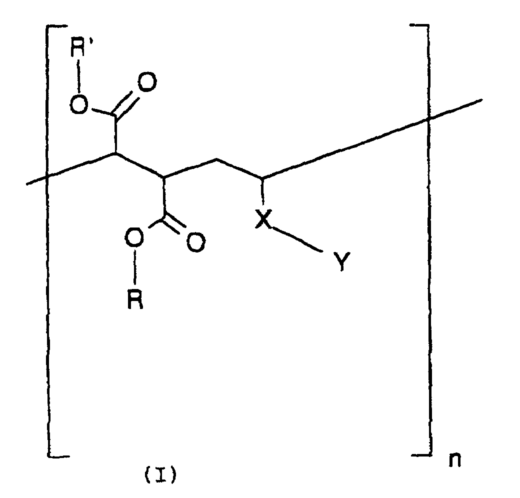 wobei
R und R' gleich oder verschieden sein können und H, Alkalimetall, Ammonium, C₁-C₃₀ Alkyl,
X eine direkte Bindung, C₁-C₈ Alkylen oder C₂-C₁₂ Oxoalkylen mit 1-6 Sauerstoffatomen,
Y ein UV-Licht absorbierender organischer Rest,
n 2-800 bedeutet.

2. Kosmetisches Mittel nach Anspruch 1, **dadurch gekennzeichnet, daß** R und R'ein verzweigter oder unverzweigter Alkylrest mit 8 - 20 C-Atomen bedeutet.

3. Kosmetisches Mittel nach Anspruch 1, wobei Y Licht der Wellenlänge 200 - 400 nm absorbiert.

4. Kosmetisches Mittel nach Anspruch 1, wobei Y folgende Bedeutung hat: 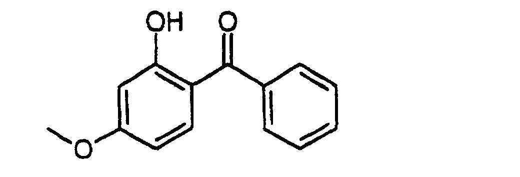 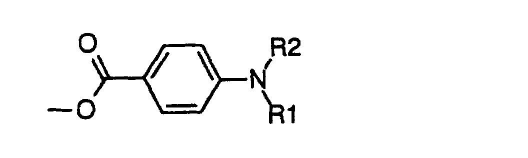 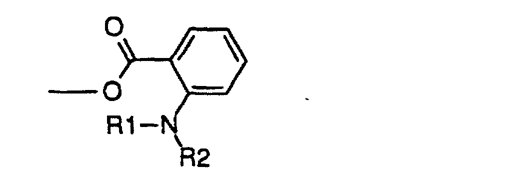 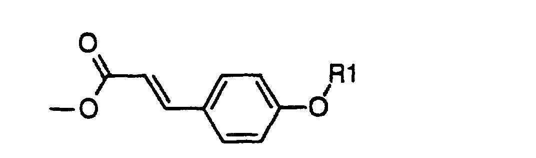 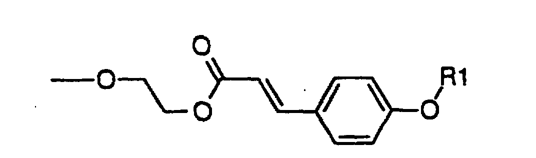 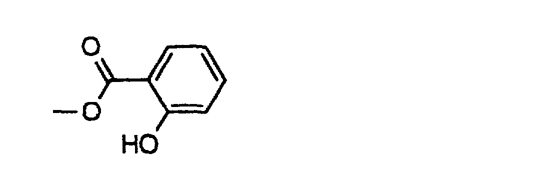 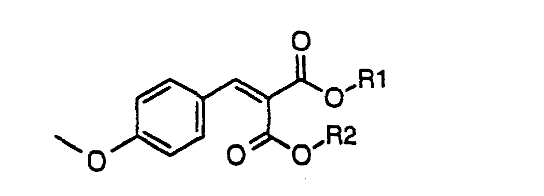 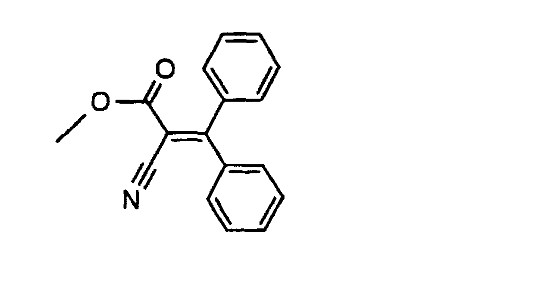 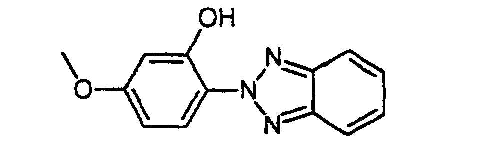 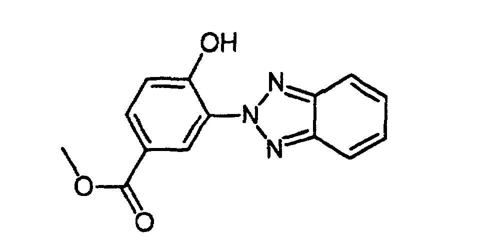 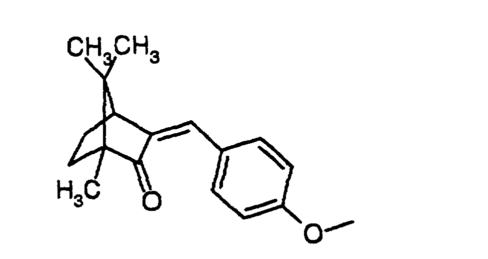 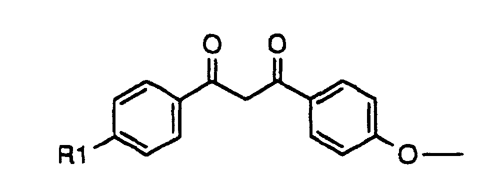 und R¹ und R² gleich oder verschieden sind, und H, C₁-C₆-Alkyl oder C₂-C₁₂ Oxoalkyl mit 1-6 Sauerstoffatomen bedeuten.

5. Verfahren zur Herstellung eines kosmetischen Mittels nach Anspruch 1, indem man
a) Maleinsäureanhydrid mit dem Monomer der Formel II 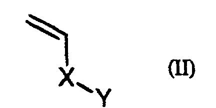 wobei X und Y die in Anspruch 1 angegebene Bedeutung haben, polymerisiert,
b) das erhaltene Polymer mit R-OH und R'-OH umsetzt, wobei R und R'die in Anspruch 1 definierte Bedeutung besitzen,
c) das nach b) erhaltene Polymer mit üblichen Hilfs- und Trägerstoffen formuliert.

## Claims

1. A cosmetic composition comprising a polymer with the repeating structural unit (I) 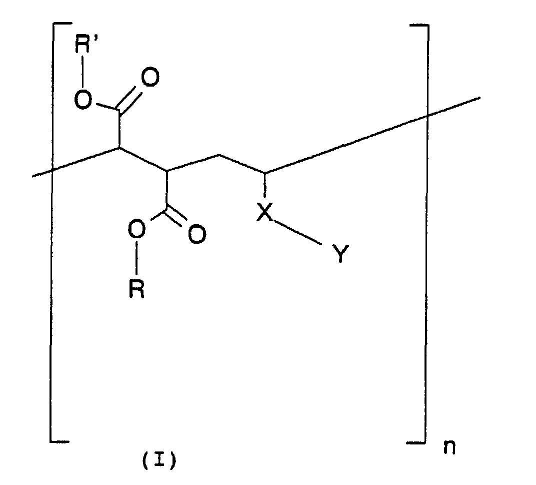 where
R and R' can be identical or different and are H, alkali metal, ammonium, C₁-C₃₀ alkyl,
X is a direct linkage, C₁-C₈ alkylene or C₂-C₁₂ oxoalkylene having 1-6 oxygen atoms,
Y is a UV-absorbing organic radical,
n is 2-800.

2. A cosmetic composition as claimed in claim 1, wherein R and R' are a branched or unbranched alkyl radical having 8 - 20 carbon atoms.

3. A cosmetic composition as claimed in claim 1, wherein Y absorbs light of the wavelength 200-400 nm.

4. A cosmetic composition as claimed in claim 1, wherein Y has the following meaning: 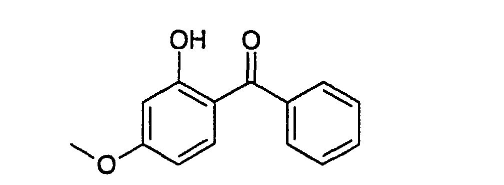 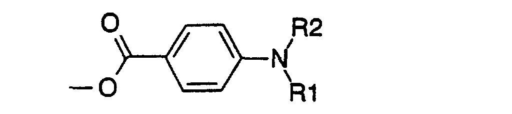 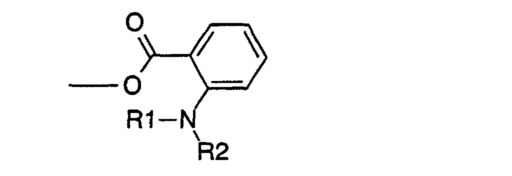 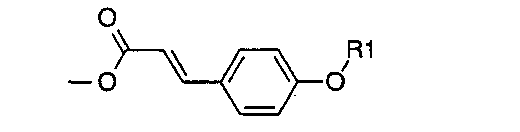 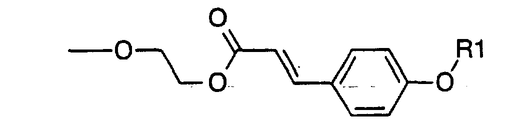 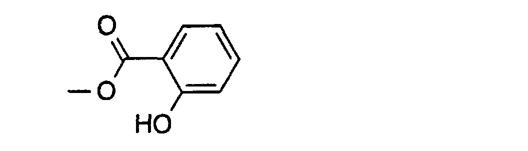 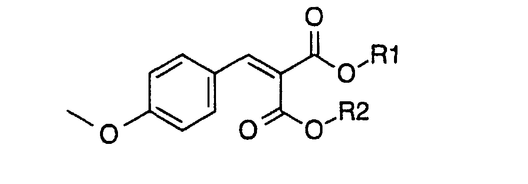 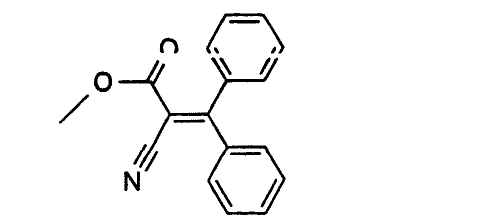 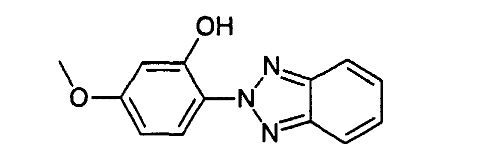 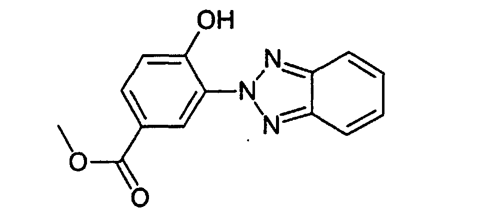 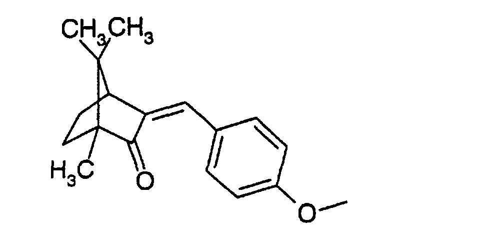 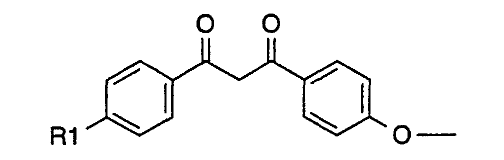 and R¹ and R² are identical or different, and are H, C₁-C₆-alkyl or C₂-C₁₂ oxoalkyl having 1-6 oxygen atoms.

5. A process for producing a cosmetic composition as claimed in claim 1, by
a) polymerizing maleic anhydride with the monomer of the formula II 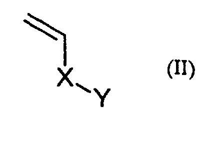 where X and Y have the meanings stated in claim 1,
b) reacting the resulting polymer with R-OH and R'-OH where R and R' have the meanings defined in claim 1,
c) formulating the polymer obtained in b) with conventional ancillary substances and carriers.

## Revendications

1. Produit cosmétique contenant un polymère à motif de structure répété I 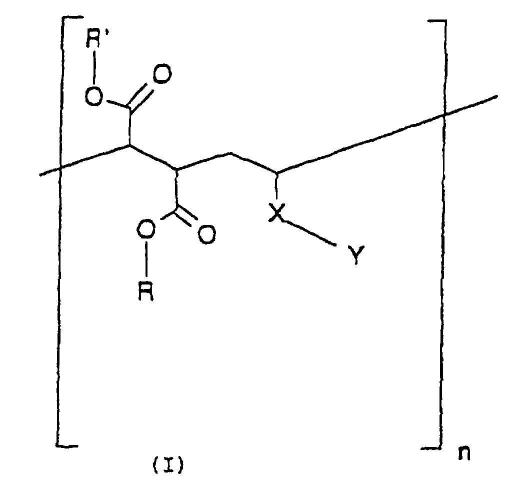 dans lequel
R et R', ayant des significations identiques ou différentes, représentent H, un métal alcalin, l'ammonium, un groupe alkyle en C1-C30,
X représente une liaison directe, un groupe alkylène en C1-C8 ou oxoalkylène en C2-C12 contenant 1 à 6 atomes d'oxygène,
Y représente un radical organique absorbant la lumière UV,
n est un nombre allant de 2 à 800.

2. Produit cosmétique selon la revendication 1, **caractérisé par le fait que** R et R' représentent des groupes alkyle à chaîne droite ou ramifiée en C8-C20.

3. Produit cosmétique selon la revendication 1, dans lequel Y absorbe la lumière de longueur d'onde 200 à 400 nm.

4. Produit cosmétique selon la revendication 1, dans lequel Y représente l'un des groupes suivants : 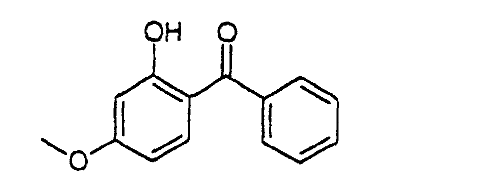 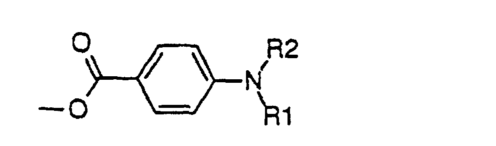 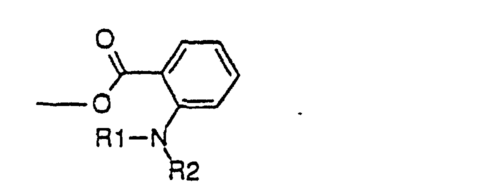 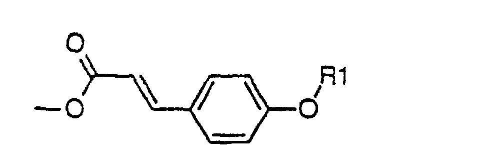 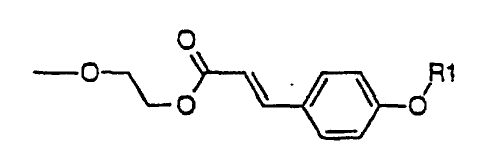 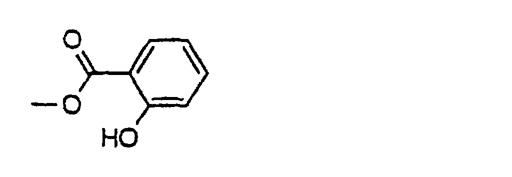 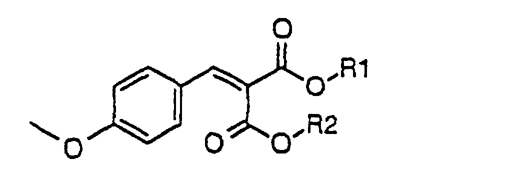 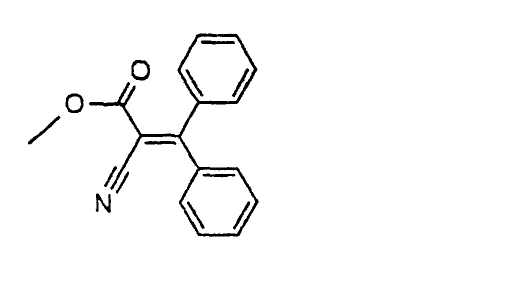 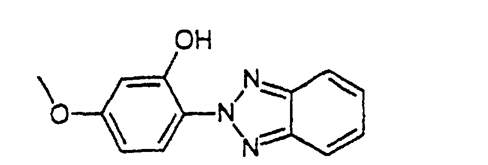 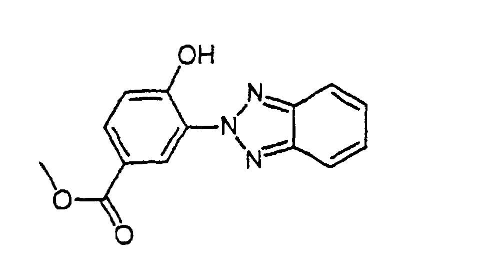 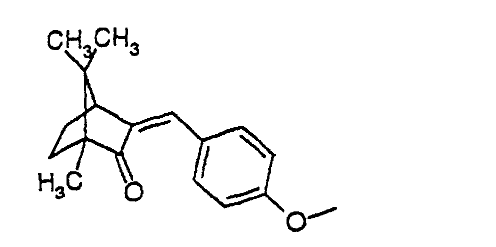 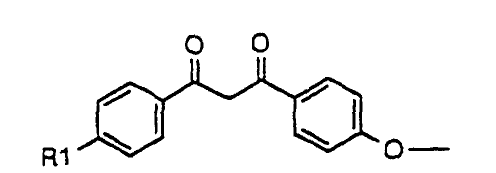 et R¹ et R², ayant des significations identiques ou différentes, représentent H, un groupe alkyle en C1-C6 ou un groupe oxoalkyle en C2-C12 contenant 1 à 6 atomes d'oxygène.

5. Procédé pour la préparation d'un produit cosmétique selon la revendication 1, selon lequel
a) on polymérise l'anhydride maléique avec le monomère de formule II 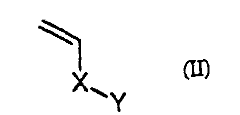 dans laquelle X et Y ont les significations indiquées dans la revendication 1,
b) on fait réagir le polymère obtenu avec R-OH et R'-OH, R et R' ayant les significations indiquées dans la revendication 1,
c) on forme le produit cosmétique avec le polymère obtenu en b) et des produits auxiliaires et véhicules usuels.
